# EUROPEAN PATENT APPLICATION

(11) **EP 1 308 472 A2**
(43) Date of publication of application: **07.05.2003**
(21) Application number: 02079200.8
(22) Date of filing: 10.10.2002
(51) Int. Cl.: C08G 18/10, A61L 15/22, C08L 75/04, A61F 13/04, A61L 15/12

(54) **Method for providing a synthetic plaster cast on a part of a body**

(30) Priority: 10.10.2001 NL 1019152
(71) Applicant: GeniMedical B.V., 3439 LC Nieuwegein (NL)
(72) Inventor: Geneuglijk, Matthijs Nicolaas, 3994 TB Houten (NL)
(74) Representative: Prins, Adrianus Willem

(57) **Abstract**

The present invention relates to a method for providing a synthetic plaster cast on a part of a body and to means with which such a method can be carried out. In particular, the invention further relates to a semi-rigid casting material obtainable by means of a method according to the invention. Also, the invention relates to a kit for carrying out a method according to the invention.

## Description

This invention relates to a method for providing a synthetic plaster cast on a part of a body and to means with which such a method can be carried out.

Providing a reinforcement on a part of a body by applying cast or tape has long since been known. In the case of the reinforcement of human or animal body parts, when treating bone fractures, it has been conventional for a long time to completely immobilize the broken body part on both sides of the fracture, first with plaster casts and later with rigid synthetic casts.

US patent 4,690,842 describes a composition with reduced tack used to reinforce a multilayered rigid plaster cast, so that the plaster becomes more resistant to damage from the outside. The plaster cast serves to immobilize body parts. The composition is a quick-foaming material that can be provided between the different bandage layers, and to which an inert thickening agent is added.

A disadvantage of the complete immobilization of a body part is, however, that the tissue, such as muscular tissue, immobilized like the bone, weakens so that after healing of the fracture and removal of the plaster cast a relatively long rehabilitation time is often necessary.

As an alternative, so-called semi-rigid synthetic casts have been used, with which weakening of tissue can be reduced. US 4,968,542 describes a composition of a casting material that can cure to a resilient, semi-rigid cured material when wound around a body part. As an example of such a curable material, an isocyanate-functional polymer is mentioned, which cures after moistening with liquid water.

Semi-rigid synthetic plaster casts, however, are not deemed suitable as such for the treatment of complex and/or unstable fractures and for the treatment of body parts that are heavily loaded after they have been provided with the cast, because semi-rigid synthetic plaster casts as such stabilize instead of immobilize, and this is generally not considered to be sufficiently reinforcing for such fractures.

US 5,913, 840 describes a method for making a formed orthopedic plaster material by winding a curable plaster cast, in particular a soft cast (SoftCast from 3M). For curing, a reinforcing component, in particular a methacrylate-containing composition, is added to the plaster cast. The plaster cast and the reinforcing component cure, which results in the formed plaster material.

EP-A 0 135 890 describes a casting material consisting of two types of fibers, in which one type of fiber shrinks and cures when exposed to heat, and the other type of fiber is an elastomer. By locally heating the casting material, a casting material can be obtained in which selective parts of the casting material are cured. To prevent play between the cured fibers, the cured fibers can be fixed by including them in a material curing to a rigid mold, such as a starch, gelatin, or a synthetic resin.

It is an object of the present invention to provide a simple new method for providing a reinforcement on a part of a body.

It has been found that this object can be achieved by a method characterized in that a part of the body is provided with a casting material and that (as a rule) on a part of that casting material a solitarily liquid curing material curing at least with the casting material to a rigid material is provided, whereby the part of the body provided with curing material is stabilized. As referred to herein, solitarily liquid means that the curing agent is liquid per se, for instance because it has the form of a liquid, a dispersion, a powder, an emulsion, and the like.

In particular, the invention relates to a method for providing a synthetic plaster cast on a part of a body, characterized in that this part of the body is provided with a casting material impregnated with an impregnating agent, that subsequently on a part (where deemed necessary) of that casting material a solitarily liquid curing agent is locally provided, and that this curing agent, at least together with the part of the casting material to which the curing agent is applied, is cured to a rigid material of curing agent and, if required, the impregnated casting material, whereby a synthetic plaster cast with at least one rigid part and with at least one semi-rigid part is formed and the part of the body provided with synthetic plaster cast is stabilized.

Figure 1 shows the result of a method according to the invention, in which the treated body part is an ankle. The curing agent (dark-colored strip) is provided here on the outside of the plaster cast (hatched).

Figure 2 shows the result of a method according to the invention, in which the treated body part is a finger. The curing agent (dark-colored strip) is provided here on the outside of the plaster cast (hatched).

With a method according to the invention, it has been found possible to stabilize a part of a body, for instance a bone with a fracture, in a simple and economically attractive manner. It turns out that no special medical knowledge is required to carry out a method according to the invention.

The solitarily liquid curing agent is applied to the outer surface of the casting material provided on the body part. The at least one rigid part is therefore substantially formed on the outside of the synthetic plaster cast. Over this rigid part, no layer needs to be applied anymore. Preferably, the casting material is therefore at least substantially free from an extra layer, in particular from an extra reinforcing layer, over at least the rigid part. More preferably, no (synthetic) plaster layer is applied over at least the rigid part.

Partly because the solitarily liquid curing agent, which, at least together with the cast forms rigid parts in or on the synthetic plaster cast, is applied (to the casting material) only after providing the casting material (on the body part to be reinforced), the step of providing a casting material is much simpler and easier in a method according to the invention than in conventional methods, such as, for instance, a method in which different types of casting materials are used, among which a number of casts cure to rigid material and a number of casts cure to a semi-rigid material.

Besides, it has been found that a plaster cast provided by means of a method according to the invention (in which the rigid layer forms a top layer of the plaster cast) has a relatively low risk of abrasion, softening, and other dermatological complications of skin covered by the plaster cast, certainly in comparison with a plaster cast in which curing agent is applied between different layers of the casting material. It is suspected that this is the result of a relatively good air and moisture permeability of a plaster cast provided according to the invention.

It has also been found that a body part stabilized by means of a method according to the invention is completely functional again after a relatively short period. By, for instance, stabilizing a broken body part by means of the invention, micro movements, which have a stimulating effect on the callus formation, can still take place in the fracture line, which can promote the healing of the fracture.

The invention can be applied to humans and animals, such as horses, cows and other cattle, and to dogs, cats, and other pets, in particular small pets, such as birds and rodents.

The invention is particularly suitable for the treatment of complex and hardly accessible fractures. From practical considerations, large parts of the body must then often be wrapped, while, in fact, only a small part of the body needs reinforcement, such as, for instance, in case of fractures in the hand or foot. Compared to known methods, the invention offers the possibility to keep the degree of impediment to the treated creature very low, while yet sufficient strength is imparted to the weak part of the body.

Also, the invention can be excellently applied to fractures in very small bones, for instance in small pets, such as birds and rodents, for instance rabbits, or bones in larger creatures, such as humans, which are located in a complex body part, such as the hand or foot, and which can hardly, if at all, be treated in the conventional manner without immobilizing a large part of the body around the fracture with a rigid plaster or rigid synthetic cast. The invention is therefore, inter alia, particularly suitable for setting fingers, toes, hand bones, foot bones, ankles, or wrists.

The impregnating agent is, in practice, a stiffening agent, for instance a resin, which, after the casting material has been provided on the body, can stiffen to a greater or lesser degree and thus contributes to the formation of a semi-rigid material.

The solitarily liquid curing agent (which, as such, is brought into liquid form) may serve as initiator and/or propagator of the curing of specific parts on and/or in the impregnated casting material, for instance by the presence of an additive, such as a catalyst or a cross-linker. Preferably, the curing agent and the impregnating agent are curable separately from each other.

Preferably, the semi-rigid casting material is at least partly cured before the solitarily liquid curing agent is applied to the casting material. More preferably, the semi-rigid casting material is cured for at least 1 minute, most preferably for 2-10 minutes, before the solitarily liquid curing agent is applied. Very good results are achieved with a method in which the casting material is cured for 3-10 minutes before the solitarily liquid curing agent is applied.

In practice, the solitarily liquid curing agent itself comprises a stiffening agent, such as a resin with which the synthetic plaster cast is locally stiffened further into a rigid material. Also, in an interaction with the impregnating agent and, if necessary, the casting material itself, the solitarily liquid curing agent can contribute to the further local stiffening of the casting material, thereby locally forming a rigid support.

In a preferred embodiment, at least the main constituent, for instance a resin, is equal in the solitarily liquid curing agent and the impregnating agent. Good results are achieved, for instance, with a method in which curing agent and impregnating agent both contain a resin which, at least after curing, is a polyurethane. In a special embodiment, the impregnating agent and the solitarily liquid curing agent have substantially the same composition, and the solitarily liquid curing agent will generally contain at least another extra curing component or curing-promoting component, such as a catalyst or cross-linker. If required, the curing agent may have a higher initial viscosity. A relatively high viscosity has an advantage in the controlled application of the curing agent. Those skilled in the art will, for that matter, simply be able to choose a suitable viscosity by way of routine on the basis of general expert knowledge and what is described herein.

It has been found that in a method in which the curing agent and the impregnating agent have substantially the same composition, a cured casting material is formed in which the rigid part is at least substantially integral with the subjacent part of the casting material, which benefits the durability of the synthetic plaster cast.

As referred to herein, a resin is a curable viscous compound or composition or cured compound or composition which comprises a polymer or a compound, such as a prepolymer, an oligomer or a monomer that is polymerizable. The resin may, for instance, comprise one or more liquid polymer/oligomer/prepolymer/monomer components or a solution of one or more polymer/oligomer/prepolymer/monomer components. As referred to herein, a polymer resin, for instance polyurethane resin, is a resin which, at least after curing, forms a material containing the relevant polymer, for instance polyurethane.

As referred to herein, stabilizing is particularly the reinforcement of a body part in case of a weak spot in that part, in particular a body part comprising a fracture, such that, on the one hand, the parts on both sides of that weak spot, at normal load, cannot excessively move with respect to each other, but that, on the other hand, still sufficient freedom of movement remains for micro movements on the weak spot, for instance in the fracture line, and/or for maintaining at least part of the muscular function (in particular the pumping function).

Thus a synthetic plaster cast can be locally provided on the outer surface with a curing agent which, at least after stiffening or curing, forms a strip, dish, or shell of a rigid material. The right place and dimensions thereof can be simply controlled by means of the invention.

As referred to herein, rigid material is particularly a material that, when used around a body part, causes such a rigidity that at least important parts of the wrapped body part, in particular parts near a weak spot (such as a fracture), are immobilized, that is to say, in these parts the muscular function is generally seriously impeded and, at normal load, no substantial micro movements take place anymore in, for instance, the fracture line.

As referred to herein, semi-rigid material is a material that, at least after curing, imparts a substantial reinforcement to the wrapped body, while, however, some movement is still possible within the wrapped body and the muscular function is maintained to a substantial extent after providing a reinforcing cast. The semi-rigid material may, for that matter, still be flexible when providing it. It need not cure until after it has been provided.

As referred to herein, a synthetic plaster cast is a synthetic-containing material, without all the components needing to be a synthetic. For the manufacture of a synthetic plaster cast, a casting material is used that generally comprises a support and an impregnating agent. The support functions as a mold of a generally fibrous material, which is, for instance, woven or otherwise formed to a tape, ribbon, bandage, cloth, strip, (orthopedic) sock (such as a knee sock, an elbow sock, an ankle sock), or to another suitable form. Depending on the form of the casting material and the body part, the casting material can be provided in a conventional manner, for instance by wrapping the body part in at least one tape, bandage, strip, ribbon, or cloth.

Suitable as (fibrous) mold materials are the conventional natural and synthetic materials, such as cotton, synthetic, or glass, which materials are often commercially available. In a preferred embodiment, the casting material comprises a polyamide, polyester, polyolefin, polyacrylamide, and/or glass fiber. A very suitable polyolefin fiber is a polypropylene fiber.

Very suitable is a casting material in which the support consists mainly of polypropylene and/or glass fiber. Especially preferred is a support having 5-20% polypropylene fiber and 95-80% glass fiber, for instance about 12% polypropylene fiber and about 88% glass fiber.

Preferably used is a semi-rigid casting material. In such a cast the support, before providing it on a part of the body, is impregnated with, for instance, a resin, such as polyurethane resin and/or a polyisocyanate, which, before, during, or after providing around the body part, can cure so that a semi-rigid system is formed. Very suitable semi-rigid casting materials are, for instance, described in US 4,968,542 and commercially available semi-rigid casts, such as Scotchcast®, Softcast (3M), or SynthoFLX™ (EBI/Biomet). Such a cast may have been or may be provided in a conventional manner. Very good results are achieved with a semi-rigid casting material on the basis of a polyurethane resin.

Preferably, an impregnated casting material is taken from a hermetically sealed package only shortly, for instance 0-5 min, before use. More preferably, the casting material is taken from a hermetically sealed package immediately before use (within about 1 min or less).

As a solitarily liquid curing agent, a composition is preferably used that comprises a polyurethane resin and/or a polyisocyanate, and more preferably a composition comprising polyurethane resin. Such a composition may serve as a resin composition curing to a rigid synthetic.

Curing the solitarily liquid curing agent and/or, locally, the impregnating agent in the casting material may take place in a conventional manner. In a preferred embodiment, curing takes place with water, for instance by moistening the cast and/or the curing agent with liquid water, for instance by immersing the bandaged body part in water, sprinkling it, or wrapping the bandaged body part in or covering it with wet bandage. Curing may, if required, also be effectively realized by exposure to (the moisture in) the air, without moistening with liquid water.

The solitarily liquid curing agent may comprise an inorganic and/or an organic solvent (such as water and/or dimorpholinodiethyl ether).

The solitarily liquid curing agent and/or the impregnating agent may comprise one or more active components, such as one or more curing-promoting agents (for instance nitrate-providing compounds, sulfide-providing compounds, aromatic diisocyanates, tertiary amines, and α-cyanoacrylic acid esters), surface-active agents (for instance alkyl cresols, such as 2,6-di-tert-butyl-p-cresol), adhesion-promoting agents, stabilizers (such as benzoyl chloride), antifoam agents, and/or breathing-promoting agents.

It has been found that an excessively foaming curing agent has a potential disadvantage with respect to the controllability with which a desired part of the synthetic plaster cast can be made rigid. It has also been found that this controllability can be increased by the presence of an antifoam agent without one or more parts of the plaster cast that are to remain semi-rigid being covered with curing agent as a result of the expansion of the material.

A method in which the curing agent - provided on the outside - and, if required, the impregnating agent contains at least one antifoam agent is especially preferred. Those skilled in the art will be able to choose a suitable antifoam agent and a suitable amount from agents reducing surface tension and/or bubble formation, depending on the other components of the curing agent, such as the employed resin.

Very suitable is, for instance, an antifoam agent selected from the group consisting of polyalkylsiloxanes - in particular polydimethylsiloxane and phenylmethylsiloxane - and hydrophobic silica in hydrocarbon oils of high molecular weight. Such antifoam agents are, inter alia, very suitable for use in a polyurethane-containing curing agent and also in a polyurethane-containing impregnating agent.

With respect to the content of antifoam agent, good results have been achieved, for instance, in the range of from 0.07 to 7.5 wt.%, based on the total weight of the curing agent.

A method using a curing agent with an antifoam agent is, partly because of the controllability of the covering operation, therefore very suitable, inter alia, for the treatment of complex and/or small body parts.

Besides, it has been found that in a method in which at least the curing agent contains an antifoam agent a very good rigidity of one or more rigid parts of the plaster cast is obtained.

One or more detackifying agents (such as silicone oil, silicone surfactant) may be used, but very good results have been achieved with a method in which at least the solitarily liquid curing agent is substantially free from such detackifying agents.

Suitable nitrate-providing compounds may be nitrate salts, for instance alkali metal nitrates (such as NaNO₃ and KNO₃) and alkaline earth metal nitrates, but other compounds are of course also suitable.

Suitable sulfide-providing compounds are sulfide salts, such as zinc sulfide, but other compounds are of course also suitable.

Examples of suitable tertiary amines are 2,2'-dimorpholinodiethyl ether and 4-[2-[1-methyl-2-(4-morpholinyl)ethoxy]ethyl]morpholine.

Preferred among the aromatic diisocyanates are diphenylalkane diisocyanates. Especially preferred are 4,4'-diphenylmethane diisocyanate, 2,4'-diphenylmethane diisocyanate, and 2,2'-diphenylmethane diisocyanate.

Very good results have been achieved with a method in which the solitarily liquid curing agent comprises a polyurethane resin, 4,4'-diphenylmethane diisocyanate, and in addition, a nitrate-providing compound and/or a sulfide-providing compound. Such a curing agent enables a very good interaction with the curing agent and the cast fabric (mold), in particular when the impregnating agent also comprises a polyurethane resin, for instance 4,4'-diphenylmethane diisocyanate. Thus a very suitable composite of polyurethane and cast fabric can be obtained.

In one embodiment the solitarily liquid curing agent also contains one or more components selected from the group formed by sulfide-providing compounds, zinc-providing compounds, alkylbenzylphthalates (for instance butylbenzylphthalate), and polyols.

It is not necessary to add to the curing agent inert (in particular non-polymerizing) thickening agents, such as those occasionally employed in the state of the art. Preferably, the curing agent and, if required, the impregnating agent is at least substantially free from such inert thickening agents, in particular inert from inorganic filling agents (such as talc and silica) and inert organic polymer powders (such as polymethylmethacrylate).

A solitarily liquid curing agent is applied to specific parts of the casting material. This casting material is first provided on the desired spot, for instance in the form of a tape-like cast, ribbon-like bandages, or strips. These can be wrapped around the body part in a conventional manner or can be provided around the body part by a cast sock or otherwise formed casting material. The solitarily liquid curing agent can be applied in various manners. Very suitable is a method in which this is done with the aid of a dosing unit, such as a sprayer (for instance a synthetic disposable sprayer), a squeeze bottle, a can, a dabber, or a bottle, flacon, or stick provided with a roller, or an equivalent type of dosing unit. Very good results have been achieved with a dosing unit hermetically sealed before use and with a disposable dosing unit.

Thus it is possible to locally impart, in a surprisingly simple manner, extra rigidity to a fracture in specific places, without unnecessarily limiting the freedom of movement of relatively large parts of the body that are also provided with cast.

The invention offers the possibility to selectively provide, in a very simple and efficient manner, independently of the size and direction of the bandages or strips of the cast, extra stiffenings on the casting material provided around the body. Thus, for instance, extra stiffenings can be provided over only a portion of the width of the bandages or strips of the casting material, or specifically at a number of crossings or overlaps of two pieces of casting material, extra rigidity can be imparted to the synthetic plaster cast to be formed.

The invention further relates to a semi-rigid casting material with rigid parts, which is obtainable by means of the invention.

Also, the invention relates to a semi-rigid synthetic plaster cast - preferably a formed semi-rigid synthetic plaster cast- which is further stiffened on some parts of the cast so that, apart from semi-rigid parts, this cast also contains rigid parts. These rigid parts particularly comprise a rigid material (such as a composite) formed by interaction of an impregnated casting material and a curing agent for further stiffening.

Depending on the application, a relatively small or a relatively large part of the casting material may be rigid. For many applications, about 2-80%, more in particular 5-60%, more preferably less than 30%, for instance 10-30%, of the outer surface of the final plaster cast will be rigid. Very good results are also achieved with a casting material in which less than 10% of the outer surface is rigid.

In one embodiment the local further stiffening in the semi-rigid synthetic plaster cast is independent of the size and orientation of the casting material.

Very good results are achieved with a semi-rigid synthetic plaster cast in which the locally further stiffened parts have the form of a strip, dish, shell, or a ring (cylinder), preferably a strip, around the outer surface of the synthetic plaster cast. Preferably, at least the inside of the strip, dish, ring, or shell extends to at least an outer part of the casting fabric, which benefits the adhesion of the strip, shell, dish, or ring to the rest of the synthetic plaster cast.

Furthermore, the invention relates to a kit for carrying out a method by means of the invention, comprising a casting material for providing a semi-rigid reinforcement on a body, and separately packaged curing agent with which the synthetic plaster cast can be locally extra stiffened so that rigid pieces can be formed.

Preferably, the separately packaged solitarily liquid curing agent is hermetically packaged. Preferably, the casting material has been impregnated and hermetically packaged previously (in the factory).

In one embodiment of a kit the separately packaged resin composition is packaged in a dosing unit, such as a sprayer, a squeeze flacon, or a container with a roller, such as a roller stick or a dabber.

In one embodiment the separately packaged resin composition is packaged in a package for single use.

Depending on the application, the separately packaged resin composition, serving as curing agent, may be packaged in different dosing units. For many applications, the packaged amount will be in the range of 1-250 ml, for instance in the range of 1-200 ml. In case of applications in which small surfaces are treated, for instance for small pets, or for the treatment of body extremities, such as fingers or toes, it may be preferred to use a smaller dosing unit, for instance 1-25 ml.

Very suitable is a kit in which the separately packaged curing agent is a polyurethane resin, and in which, if required, the casting material is also impregnated previously and contains a polyurethane resin.

Very good results have further been achieved with a kit in which the separately packaged solitarily liquid curing agent contains an aromatic diisocyanate and, furthermore, at least one nitrate-providing compound, for instance a sodium salt, and/or at least one sulfide-providing compound, for instance a sulfide salt, such as zinc sulfide.

The invention further relates to a solitarily liquid curing agent, if required in a kit according to the invention, which, at least together with the casting material, is curable to a rigid material, for instance a rigid composite, which curing agent comprises a resin, preferably polyurethane resin, a diphenylmethane diisocyanate, preferably 4,4'-diphenylmethane diisocyanate, and nitrate and/or sulfide.

A solitarily liquid curing agent according to the invention may also be used for the manufacture of a liquid agent that can be used in the treatment of a fracture.

The invention also relates to a hermetically sealed disposable package, comprising a solitarily liquid curing agent suitable for use by means of the invention, and in particular a disposable package provided with a dosing unit for applying the curing agent to a casting material.

Very suitable is a hermetically sealed disposable package in the form of a squeeze bottle, flacon, dabber, roller stick, or a bottle with a roller.

A kit or a curing agent according to the invention has been found very suitable for use in the stabilization of a fracture, such as leg fractures, arm fractures, ankle fractures, elbow fractures, and fractures in the hand (for instance of a finger) or the foot.

The invention will now be illustrated in more detail with reference to a number of figures and formulation examples.

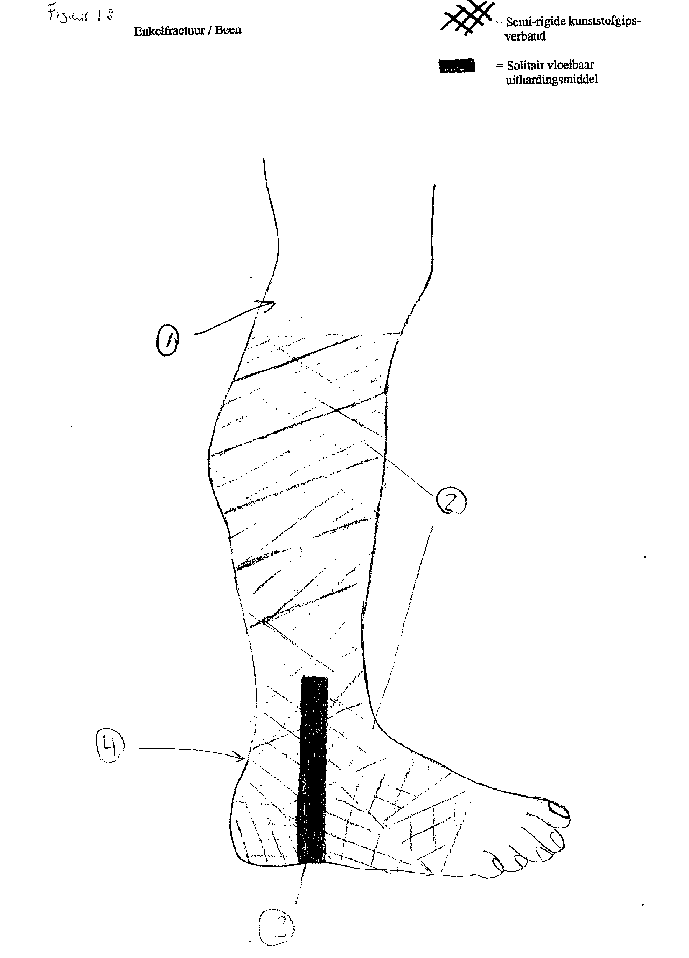

Figure 1 shows an embodiment for the treatment of an ankle fracture, in which a leg (1) and ankle (4) are wrapped in a semi-rigid casting material (2), on which a U-shaped strip (3) of the solitarily liquid curing agent is provided around the ankle over one side of the ankle, the lower side of the foot, and over the other side of the ankle (only one side is shown).

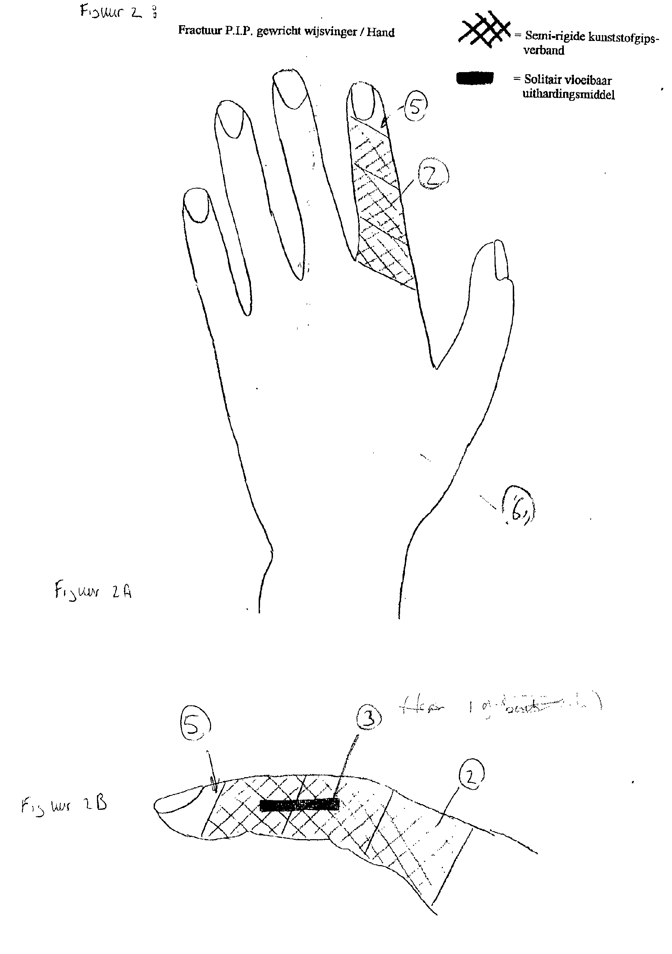

Figure 2A shows an embodiment for the treatment of a fracture in the joint of a finger. The index finger (5) on the hand (6) is wrapped in a semi-rigid casting material (2). Figure 2B shows how a strip with solitarily liquid curing agent (3) is provided on one of the sides of the index finger (5). Optionally, solitarily liquid curing agent may also be provided on the other side.

### Example 1

Formulation example for a polyurethane resin composition curing to a rigid synthetic:

| Component | parts by weight |
|---|---|
| 4,4'-diphenylmethane diisocyanate polypropylene glycol | 30-35 |
| diphenylmethane diisocyanate homopolymer | 0-3 |
| 4,4'-diphenylmethane diisocyanate | 3-8 |
| Zinc sulfide | 0-3 |
| 4-[2-[1-methyl-2-(4-morpholinyl)ethoxy]ethyl]morpholine | 0.1-2 |
| butylbenzylphthalate | 0-2 |
| 2,6-di-tert-butyl-p-cresol | 0-0.05 |
| benzoyl chloride | 0-0.05 |
| colorant | 0-2 |

### Example 2

Formulation example for a polyurethane resin composition curing to a rigid synthetic:

| Component | parts by weight |
|---|---|
| 4,4'-diphenylmethane diisocyanate polypropylene glycol | 30-35 |
| diphenylmethane diisocyanate homopolymer | 0-3 |
| nitrate | 0-5 |
| 4,4'-diphenylmethane diisocyanate | 3-8 |
| 4-[2-[1-methyl-2-(4-morpholinyl)ethoxy]ethyl]morpholine | 0.1-2 |
| butylbenzylphthalate | 0-2 |
| 2,6-di-tert-butyl-p-cresol | 0-0.05 |
| benzoyl chloride | 0-0.05 |
| colorant | 0-2 |

## Claims

1. A method for providing a synthetic plaster cast on a part of a body, **characterized in that**
- said part of the body is provided with a casting material impregnated with an impregnating agent, at least curing to a semi-rigid casting material,
- subsequently, on a part of said casting material a solitarily liquid curing agent is locally provided, and said solitarily liquid curing agent, at least together with the part of the casting material to which the solitarily liquid curing agent is applied, cures to a rigid material of solitarily liquid curing agent and, if required, the part of the impregnated casting material to which the curing agent is applied,
- whereby a synthetic plaster cast with at least one rigid part and with at least one semi-rigid part is formed and the part of the body provided with synthetic plaster cast is stabilized.

2. A method according to claim 1, wherein the casting material comprises a polyamide, polyester, polyolefin (for instance polypropylene), polyacrylamide, and/or glass fiber.

3. A method according to claim 2, wherein the casting material comprises polypropylene fiber and glass fiber.

4. A method according to any one of the preceding claims, wherein the solitarily liquid curing agent and/or the impregnating agent comprises a polyurethane resin and/or a polyisocyanate resin.

5. A method according to any one of the preceding claims, wherein the solitarily liquid curing agent and the impregnating agent have at least substantially the same composition.

6. A method according to any one of the preceding claims, wherein the curing agent is cured by treatment with water or in the air.

7. A method according to any one of the preceding claims, wherein the curing agent contains one or more curing-promoting agents, surface-active agents, antifoam agents, adhesion-promoting agents, stabilizers, and/or breathing-promoting agents.

8. A method according to claim 7, wherein the curing agent contains at least one antifoam agent selected from the group consisting of polyalkylsiloxanes (for instance polydimethylsiloxane, phenylmethylsiloxane) and hydrophobic silica in hydrocarbon oils of high molecular weight.

9. A method according to claim 8, wherein the antifoam agent is provided in the curing agent in a concentration of from 0.07 to 7.5 wt.%, based on the total weight of the curing agent.

10. A method according to any one of claims 7-9, wherein at least one curing-promoting agent is selected from the group consisting of nitrate-providing compounds, sulfide-providing compounds, aromatic diisocyanates, and α-cyanoacrylic acid esters.

11. A method according to claim 10, wherein the aromatic diisocyanate is a diphenylalkane diisocyanate, preferably a diphenylmethane diisocyanate.

12. A method according to any one of the preceding claims, wherein the solitarily liquid curing agent is applied by means of a sprayer, a squeeze bottle, a flacon, a roller stick, a can, or a dabber.

13. A method according to any one of the preceding claims, wherein the solitarily liquid curing agent and, if required, the impregnated casting material are taken from a hermetically sealed package shortly before use.

14. A method according to any one of the preceding claims, wherein the solitarily liquid curing agent is dispensed from a dosing unit for single use.

15. A method according to any one of the preceding claims, wherein the casting material is applied by wrapping the part of the body in the casting material.

16. A method according to any one of the preceding claims, wherein the semi-rigid casting material is at least partly cured before the solitarily liquid curing agent is applied to the casting material.

17. A method according to claim 16, wherein the semi-rigid casting material provided on the part of the body is cured for at least 1 minute, preferably for at least 2 minutes, more preferably for 3-10 minutes, before the solitarily liquid curing agent is applied.

18. A method according to any one of the preceding claims, wherein the curing agent is provided on 2-80%, preferably on 2-60%, more preferably on 2-30%, in particular on 2-10% of the outer surface of the semi-rigid casting material provided on the part of the body.

19. A semi-rigid casting material obtainable by means of a method according to any one of the preceding claims.

20. A semi-rigid synthetic plaster cast locally stiffened further into a rigid material by interaction of an impregnated casting material and a solitarily liquid curing agent for further stiffening.

21. A semi-rigid synthetic plaster cast according to claim 19 or 20, wherein the local further stiffening is independent of the size and orientation of the casting material.

22. A semi-rigid synthetic plaster cast according to any one of claims 19-21, wherein the local further stiffening is located on the outer surface of the plaster cast as a strip, ring, dish, or shell.

23. A semi-rigid synthetic plaster cast according to any one of claims 19-22, wherein 2-80%, preferably 5-60%, more preferably about 10-30% of the outer surface of the synthetic plaster cast is rigid.

24. A kit for carrying out a method according to any one of claims 1-18, comprising a casting material for providing a reinforcement on a body, and a separately packaged solitarily liquid curing agent.

25. A kit according to claim 24, wherein said separately packaged solitarily liquid curing agent and, if required, the casting material is hermetically sealed.

26. A kit according to claim 24 or 25, wherein said separately packaged solitarily liquid curing agent is packaged in a dosing unit in the form of a sprayer, a squeeze bottle, a flacon, a can, a dabber, or a bottle or stick with a roller.

27. A kit according to any one of claims 21-26, wherein said separately packaged solitarily liquid curing agent is packaged in a dosing unit for single use.

28. A kit according to any one of claims 21-27, wherein said separately packaged solitarily liquid curing agent is packaged in a dosing quantity of 1-250 ml.

29. A kit according to any one of claims 21-28, wherein said separately packaged solitarily liquid curing agent comprises a polyurethane resin.

30. A kit according to any one of claims 21-29, wherein said separately packaged solitarily liquid curing agent contains an aromatic diisocyanate and at least one nitrate-providing compound and/or sulfide-providing compound.

31. A kit according to any one of claims 21-30, wherein the casting material comprises a polypropylene fiber and/or a glass fiber.

32. A kit according to any one of claims 21-31, wherein the casting material contains a polyurethane resin.

33. A kit according to any one of claims 21-32, wherein the impregnating agent with which the casting material is impregnated and the curing agent have at least substantially the same composition.

34. A curing agent suitable for a method according to any one of claims 1-18, in the form of a resin composition, which, on a casting material, at least together with the casting material, is curable to a rigid material of synthetic and casting material, comprising a polyurethane resin, a diphenylmethane diisocyanate, and also at least one nitrate compound and/or sulfide compound.

35. A hermetically sealed disposable package having packaged therein a solitarily liquid curing agent according to claim 34 or a solitarily liquid curing agent for use in a method according to any one of claims 1-18.

36. A hermetically sealed disposable package according to claim 35, provided with a dosing unit for applying the curing agent to a casting material.

37. A hermetically sealed disposable package according to claim 36, in the form of a can, squeeze bottle, flacon, roller stick, dabber, or a bottle with a roller.

38. The use of a kit according to any one of claims 24-33, a solitarily liquid curing agent according to claim 34, or a hermetically sealed disposable package according to claim 35, 36 or 37 for stabilizing a fracture.

39. The use of a solitarily liquid curing agent according to claim 34, for the preparation of a liquid agent for the treatment of a fracture.
